# FASCICULE DE BREVET EUROPEEN

(11) **EP 2 790 608 B1**
(45) Date de publication et mention de la délivrance du brevet: **01.09.2021**
(21) Numéro de dépôt: 12816688.1
(22) Date de dépôt: 10.12.2012
(51) Int. Cl.: A61F 2/07

(54) **PROTHÈSE ENDOVASCULAIRE**
ENDOVASKULÄRE PROTHESE
ENDOVASCULAR PROSTHESIS

(30) Priorité: 15.12.2011 FR 1103867
(43) Date de publication de la demande: 22.10.2014
(73) Titulaire: Assistance Publique Hôpitaux De Paris, 75004 Paris (FR); Université Pierre et Marie Curie, 75005 Paris (FR)
(72) Inventeur: KOSKAS, Fabien, F-75011 Paris (FR); MOLINA, Julien, F-75005 Paris (FR); CHO, Chika, F-75012 Paris (FR)
(74) Mandataire: Palacci, Jeremie
(86) Numéro de dépôt international: PCT/FR2012/000510
(87) Numéro de publication internationale: WO 2013/088004

(56) Documents cités:
- WO-A1-2009/020653
- WO-A1-2010/024867
- US-B1- 6 645 242

## Description

La présente invention se rapporte à une prothèse endovasculaire.

Les prothèses endovasculaires sont des dispositifs destinés à être positionnés à l'intérieur de vaisseaux sanguins pour traiter des lésions anévrismales notamment au niveau de l'aorte.

Ces prothèses sont généralement de forme tubulaire et comprennent classiquement une structure de soutien rigide (généralement métallique) communément connue sous le nom de « *stent* », recouverte d'un tissu synthétique. Ces prothèses sont encore connues sous le nom d'endoprothèses vasculaires ou « *stent-grafts* ». Le tissu recouvrant le soutien rigide est choisi pour se substituer au moins partiellement à la paroi vasculaire.

Il est connu que chez l'être humain des anévrismes peuvent porter atteinte à la santé. Il s'agit de la formation d'une ou plusieurs cavités locales (ou poches) au niveau de la paroi d'une artère ou d'une veine. Ces cavités se remplissent de sang tout en communiquant avec ladite artère ou veine. Un risque majeur lié à la formation de ces cavités est la possibilité de leur rupture. On parle alors de rupture de l'anévrisme qui peut entrainer une hémorragie interne. Une hémorragie interne est un événement dangereux qui peut entraîner la mort par exsanguination.

Dans la technique, un anévrisme peut être défini comme la perte de parallélisme de paroi d'un vaisseau. En d'autres termes, les parois sont dilatées et se comportent comme une poche de moindre résistance mais tout de même exposée à la pression sanguine. Comme mentionné ci-dessus, la complication principale des anévrismes est la rupture qui conduit au décès dans plus de 80 % des cas.

Ainsi, le traitement par prothèse endovasculaire vise essentiellement à préserver une circulation sanguine intacte. Le principe du traitement est d'exclure la cavité anévrismale du flux sanguin. Ceci est réalisé en positionnant une prothèse endovasculaire dans l'artère ou veine lésée au niveau de la cavité anévrismale. Plus précisément, la prothèse endovasculaire est disposée pour épouser la paroi interne de l'artère ou veine lésée, et ce au niveau de la jonction vaisseau sanguin-cavité anévrismale. Ainsi, on crée une étanchéité du vaisseau sanguin vis-à-vis de la cavité anévrismale.

La prothèse endovasculaire est interposée entre l'artère saine d'amont et l'artère saine d'aval ou respectivement collet proximal et collet distal. La prothèse endovasculaire forme alors un canal étanche qui assure la fonction de vaisseaux artificiel tout en excluant la cavité anévrismale.

Une prothèse endovasculaire est introduite par des techniques connues d'angioplastie. Parmi ces techniques, on peut notamment citer le cathétérisme ou la navigation artérielle. Ces techniques permettent d'éviter la chirurgie conventionnelle dite « chirurgie ouverte » qui est plus invasive et qui nécessite souvent une ouverture du thorax ou de l'abdomen.

Les prothèses endovasculaires classiques permettent ainsi de traiter les anévrismes siégeant à distance de zones de naissance de collatérales. Mais, les anévrismes siégeant à proximité de zones de naissance de collatérales (tout particulièrement proches de collatérales d'importance vitale ou fonctionnelle) posent davantage de difficultés à traiter. En effet, le traitement par prothèse endovasculaire doit conserver l'irrigation (ou la perfusion) intacte des collatérales.

Les anévrismes de l'aorte font partie des troubles qui peuvent être traités au moyen de prothèses endovasculaires. Toutefois, l'aorte présente une anatomie complexe, et ce notamment à l'origine de collatérales essentielles comme le tronc artériel brachio-céphalique qui dont le flux sanguin qui irrigue la tête doit être préservé. Un traitement d'un anévrisme de l'aorte par prothèses endovasculaires doit donc préserver une irrigation sanguine intacte au niveau de chaque zone de bifurcation.

Ceci n'est que difficilement réalisable. De plus, chaque patient présente une anatomie qui lui est propre. Les prothèses endovasculaires connues dans l'art sont ainsi généralement fabriquées sur mesure en tenant compte de l'anatomie du patient.

Les prothèses de l'état de la technique présentent un encombrement stérique qui rend difficile leur manipulation. Il s'ensuit des difficultés lors de leur positionnement dans le corps d'un patient, et notamment au niveau de courbures artérielles importantes.

La publication scientifique « Fenestrated and branched devices in the pipeline; Greenberg and Qureshi; Journal Of Vascular Surgery, Volume 52, Number 13S, 2010 » divulgue des prothèses endovasculaires connues dans l'état de la technique.

Le document WO 2009/102441 A1 décrit une prothèse endovasculaire apte à être placée dans une région courbée de l'aorte. La prothèse ne prévoit pas de bifurcation.

Le document WO 2008/107885 A2 décrit un système de prothèse endovasculaire prévu pour gérer une bifurcation au niveau de l'aorte.

Le document WO 2011/070576 A1 décrit un système de prothèse endovasculaire prévu pour gérer un croisement au niveau de l'aorte. Le document US6645242 montre un stent intravasculaire bifurqué comprenant des segments de stent primaires et un manchon de greffe primaire, formant un canal de fluide principal et ayant une ouverture latérale à travers ceux-ci.

La présente invention vient améliorer la situation.

À cet effet, l'invention propose une prothèse endovasculaire comprenant une première armature expansible et une première chemise pour ladite première armature, ladite première armature et ladite première chemise formant un premier canal lorsque la première armature est à l'état expansé. La première armature et la première chemise présentent chacune au moins une ouverture disposées sensiblement en regard l'une de l'autre et à travers lesquelles un manchon est reçu, ledit manchon présentant une première extrémité et une deuxième extrémité, et dont le pourtour de ladite première extrémité est solidaire avec le pourtour de l'ouverture de la première chemise tandis que ladite deuxième extrémité s'étend à l'intérieur dudit premier canal, et en ce que chaque manchon est dépourvu d'armature métallique et pend librement à l'intérieur du canal.

La prothèse endovasculaire de l'invention présente une architecture qui permet notamment de gérer des bifurcations rencontrées dans l'anatomie. Comme il sera décrit plus loin, chaque manchon présente une double fonctionnalité qui est à l'origine d'une liberté de manipulation, à la fois lors de la production et lors du positionnement de ladite prothèse.

La première armature et la première chemise peuvent présenter chacune plusieurs ouvertures disposées sensiblement en regard les unes des autres et à travers lesquelles un manchon est respectivement reçu. Chaque manchon présente alors une première extrémité et une deuxième extrémité, et chaque pourtour des premières extrémités est solidaire avec chaque pourtour des ouvertures de la première chemise tandis que chaque deuxième extrémité du manchon s'étend à l'intérieur dudit premier canal.

La prothèse endovasculaire peut comprendre en outre une deuxième armature expansible et une deuxième chemise pour ladite deuxième armature. La deuxième armature et la deuxième chemise forment un deuxième canal lorsque la deuxième armature est à l'état expansé. Dans ce mode de réalisation le deuxième canal débouche dans ledit premier canal à travers un manchon correspondant, tandis que ce manchon épouse au moins partiellement le pourtour extérieur dudit deuxième canal pour former une bifurcation étanche.

La prothèse endovasculaire peut comprendre en outre une troisième armature expansible disposée à l'intérieur du premier canal au niveau des ouvertures à travers lesquelles un manchon est reçu, ladite troisième armature et le manchon formant un opercule pour lesdites ouvertures lorsque la troisième armature est à l'état expansé.

Chaque armature peut être réalisée en une matière métallique comprenant un acier inoxydable biocompatible, et de préférence comprenant un matériau radio-opaque et/ou biocompatible.

La chemise peut être réalisée en une matière tissée, de préférence une matière tissée en polyester. La chemise peut aussi être réalisée comprendre une membrane poreuse, de préférence une membrane en poly-tetra-fluoro-éthylène (PTFE)..

La chemise présente de préférence une épaisseur inférieure ou égale à 0,4 millimètre.

Chaque manchon peut être réalisé en une matière sensiblement identique à celle de chaque chemise.

Dans un mode de réalisation la prothèse endovasculaire présente un premier canal d'une longueur comprise entre 30mm et 250mm et d'un diamètre compris entre 10mm et 50mm. Dans ce mode de réalisation, chaque manchon présente une longueur comprise entre 10mm et 150mm (par exemple 5mm et 30mm) et un diamètre compris entre 6mm et 20mm.

La première armature expansible peut comprendre une grille métallique expansible. Dans ce mode de réalisation la première chemise est fixée à cette grille métallique expansible par des points de couture. La chemise peut être fixée du côté intérieur ou extérieur de la grille.

Dans un mode de réalisation le pourtour de chaque première extrémité de manchon est solidarisé par couture respectivement avec chaque pourtour d'ouverture de la première chemise correspondant.

L'invention vise également un kit pour prothèse endovasculaire comprenant :
a) une première armature expansible et une première chemise pour ladite première armature, ladite première armature et ladite première chemise étant agencées pour former un premier canal lorsque la première armature est à l'état expansé,
b) une deuxième armature expansible et une deuxième chemise pour ladite deuxième armature ladite deuxième armature et ladite deuxième chemise étant agencées pour former un deuxième canal lorsque la deuxième armature est à l'état expansé.

Dans ce kit la première armature et la première chemise présentent chacune au moins une ouverture disposées sensiblement en regard l'une de l'autre et à travers lesquelles un manchon est reçu, ledit manchon présentant une première extrémité et une deuxième extrémité, et dont le pourtour de ladite première extrémité est solidaire avec le pourtour de l'ouverture de la première chemise tandis que ladite deuxième extrémité s'étend à l'intérieur dudit premier canal, et en ce que chaque manchon est dépourvu d'armature métallique et pend librement à l'intérieur du canal.

Le kit peut en outre comprendre une troisième armature expansible prévue pour être disposée à l'intérieur dudit premier canal au niveau des ouvertures à travers lesquelles un manchon est reçu.

D'autres avantages et caractéristiques de l'invention apparaîtront à la lecture de la description détaillée ci-après et sur les dessins annexés sur lesquels :
- la figure 1 représente une vue de face d'une première armature expansible de la prothèse endovasculaire de l'invention,
- la figure 2 représente une vue de côté de la première armature expansible de la figure 1,
- la figure 3 représente une vue de face de la première armature expansible de la figure 1 munie d'une première chemise de la prothèse endovasculaire de l'invention,
- la figure 4 représente une vue de côté de la première armature expansible munie d'une première chemise de la prothèse endovasculaire de la figure 3,
- la figure 5 représente une vue en perspective d'une prothèse endovasculaire de l'invention en état courbé,
- la figure 6 représente une vue de face d'une deuxième armature expansible de la prothèse endovasculaire selon un mode de réalisation de l'invention,
- la figure 7 représente une vue de face de la deuxième armature expansible de la figure 6 munie d'une deuxième chemise de la prothèse endovasculaire selon un mode de réalisation de l'invention,
- la figure 8 représente une vue de côté de la deuxième armature expansible munie d'une deuxième chemise de la prothèse endovasculaire de la figure 7,
- la figure 9 représente une vue en perspective d'une prothèse endovasculaire selon un mode de réalisation de l'invention,
- la figure 10 représente vue de face d'une troisième armature expansible de la prothèse endovasculaire selon un mode de réalisation de l'invention,
- la figure 11 représente une vue en perspective d'une prothèse endovasculaire munie d'une troisième armature expansible selon un autre mode de réalisation de l'invention, et
- la figure 12 représente une vue de côté de la prothèse endovasculaire munie d'une troisième armature expansible selon un autre mode de réalisation de l'invention.

Les dessins et la description ci-après contiennent, pour l'essentiel, des éléments de caractère certain. Les dessins représentent, pour partie au moins, des aspects difficiles à décrire autrement que par le dessin. Ils font partie intégrante de la description, et pourront donc non seulement servir à mieux faire comprendre la présente invention, mais aussi contribuer à sa définition, le cas échéant.

La présente invention prévoit un assemblage de plusieurs pièces de structure et d'architecture particulières pour éviter les inconvénients de l'état de la technique. De manière générale, la prothèse endovasculaire de l'invention comprend un canal principal muni d'éléments de bifurcation. Selon un mode de réalisation, chaque élément de bifurcation permet un raccord étanche vers un autre canal pour gérer les bifurcations rencontré dans l'anatomie. Selon un autre mode de réalisation, chaque élément de bifurcation permet une obturation étanche du canal principal. En d'autres termes, chaque élément de bifurcation peut, au choix, être raccordé vers un autre canal ou être obturé pour étanchéifier le canal principal.

Pour cela, la prothèse endovasculaire prévoit notamment un ou plusieurs manchons qui s'étendent à l'intérieur du canal principal. Le dimensionnement de chaque manchon est choisi pour assurer sa double fonction bifurcation-obturation. Le dimensionnement est aussi choisi en fonction du diamètre du canal principal.

Comme mentionné plus haut, la structure et architecture de la prothèse endovasculaire de l'invention prévoit des particularités qui sont décrits maintenant en référence aux dessins.

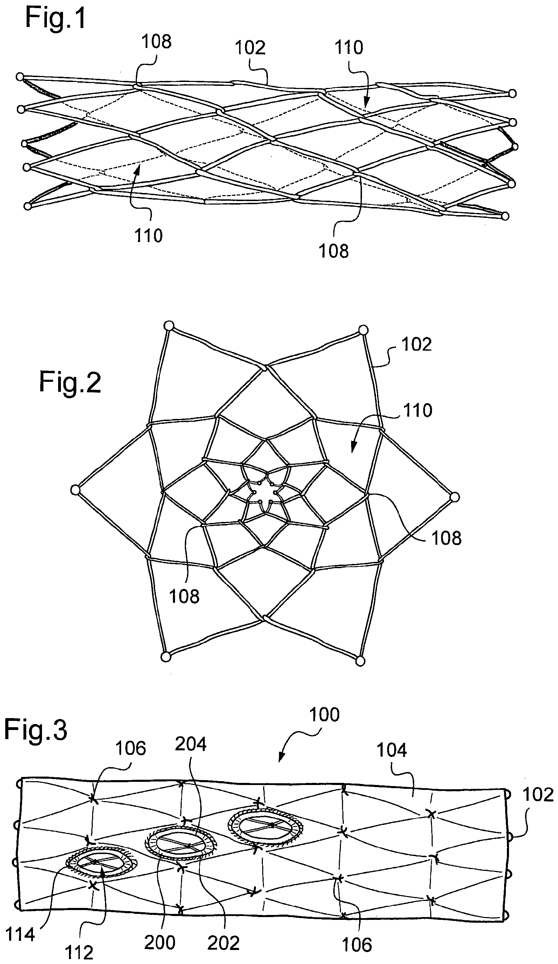

La figure 1 représente une vue de face d'une première armature expansible 102 de la prothèse endovasculaire de l'invention. L'armature 102 est représentée à l'état expansé. Elle est formée par un maillage en un matériau métallique. Le maillage peut être réalisé par tressage ou entrecroisement de fils métalliques. Il s'ensuit une structure maillée présentant des nœuds 108 au niveau de l'entrecroisement desdits fils. L'aspect général de la première armature expansible 102 est tubulaire, comme il peut être vu plus précisément sur la figure 2 qui représente une vue de côté de la première armature expansible 102 de la figure 1. La figure 2 montre également que la première armature expansible 102 défini un espace intérieur vide.

Le maillage du mode de réalisation des figures 1 et 2 présente une structure régulière formée par une suite de polygones et plus précisément par une suite de losanges. Les polygones étant formés par des fils métalliques sont vides. En conséquence, chaque polygone comprend une ouverture 110.

Le matériau métallique utilisé pour construire la première armature expansible 102 est au moins partiellement flexible. Ainsi, l'armature 102 peut être compressée sur toute sa longueur pour être enfilée dans un cathéter et permettre le positionnement dans le corps d'un patient.

Le matériau de la première armature expansible 102 peut être de l'acier inoxydable. Le matériau est choisi biocompatible et comporte de préférence un matériau radio-opaque pour favoriser la manipulation lors du positionnement de la prothèse chez un patient ainsi que pour permettre une analyse post-opératoire. En effet, une prothèse comprenant un matériau radio-opaque permet une identification précise lors d'une imagerie médicale à rayonnement électromagnétique de type rayons X (radiographie). On peut notamment prévoir un matériau de type alliage cobalt-chrome.

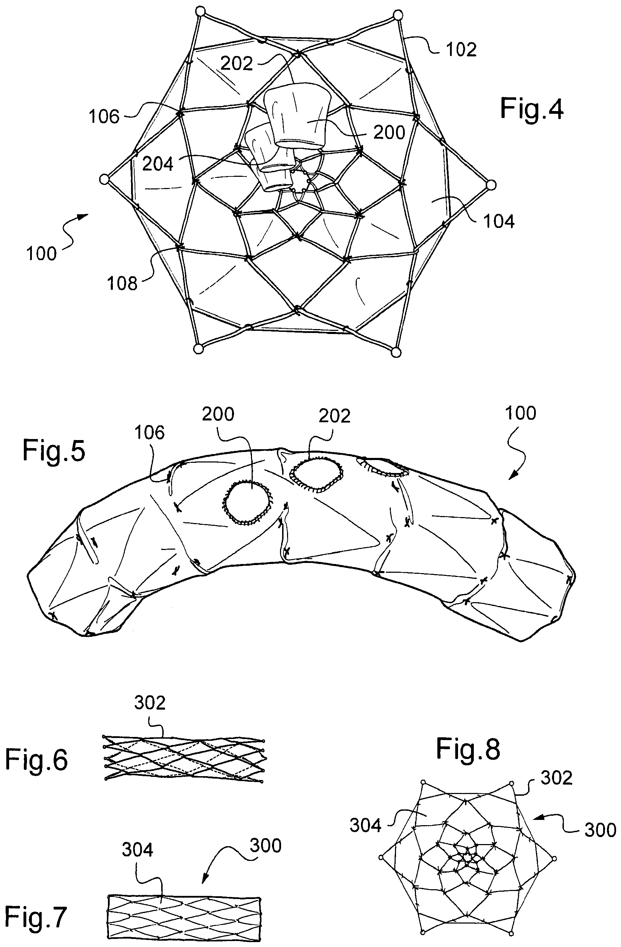

La figure 3 représente une vue de face de la première armature expansible 102 munie d'une première chemise 104. La chemise 104 est attachée sur le côté extérieur de l'armature 102 par des points de couture 106 réalisés au niveau des nœuds 108. Ainsi, la première armature expansible 102 ensemble avec la première chemise 104 forment un premier canal 100. La figure 4 montre une vue de côté de ce premier canal 100. La chemise 104 est réalisée en une matière tissée, de préférence une matière tissée en polyester.

La première chemise 104 comprend au moins une ouverture 112. Chaque ouverture 112 de la première chemise 104 est réalisée par une découpe de ladite chemise 104. Ainsi, chaque ouverture 112 de la première chemise 104 est délimitée circonférentiellement par un pourtour 114 de l'ouverture 112 de la première chemise. Le pourtour 114 de l'ouverture de la première chemise peut notamment présenter une forme circulaire, elliptique ou ovoïde. De manière générale une forme circulaire est préférée.

Chaque ouverture 112 de la première chemise est disposée sensiblement en regard d'une ouverture 110 de la première armature expansible 102. Un manchon 200 est reçu à travers chaque ouverture respective 110, 112. Le manchon 200 présente une première extrémité 202 et une deuxième extrémité 204. Le pourtour de ladite première extrémité 202 est solidaire avec le pourtour 114 de l'ouverture 112 de la première chemise 104. Dans un mode de réalisation la première extrémité 202 est cousue le long du pourtour 114 de l'ouverture 112 de la première chemise 104. La deuxième extrémité 204 s'étend à l'intérieur du premier canal 100.

Chaque manchon 200 est réalisé en une matière tissée analogue à celle de la première chemise 104 par exemple en polyester.

La figure 5 montre une vue en perspective d'une prothèse endovasculaire de l'invention et montre le canal 100 en état courbé. La première armature expansible 102 est au moins partiellement flexible afin de permettre à la prothèse de s'adapter selon l'anatomie du vaisseau sanguin. Le canal 100 formé par l'armature 102 recouverte par sa chemise 104 peut ainsi épouser la paroi interne du vaisseau sanguin dans lequel il est introduit. Chaque manchon 200 peut être disposé en vis-à-vis d'une bifurcation de vaisseau sanguin ou pas.

La figure 6 représente une vue de face d'une deuxième armature expansible 302 de la prothèse endovasculaire selon un mode de réalisation de l'invention. La structure et l'architecture sont comparables à la première armature expansible 102. En effet, il est prévu une réalisation par tressage ou entrecroisement par fils métallique en un matériau analogue à celui de la première armature 102.

Toutefois, le dimensionnement de la deuxième armature expansible 302 est réduit par rapport à celui de la première armature expansible 102. Ainsi, le dimensionnement de la deuxième armature expansible 302 est choisi pour former un deuxième canal 300 de diamètre réduit par rapport au premier canal 100, comme il est montré sur les figures 7 et 8. La figure 7 montre une vue de face de la deuxième armature expansible 302 munie d'une deuxième chemise 304 qui forment ensemble le deuxième canal 300 de la prothèse endovasculaire selon un mode de réalisation de l'invention. La figure 8 montre une vue de côté de ce deuxième canal 300.

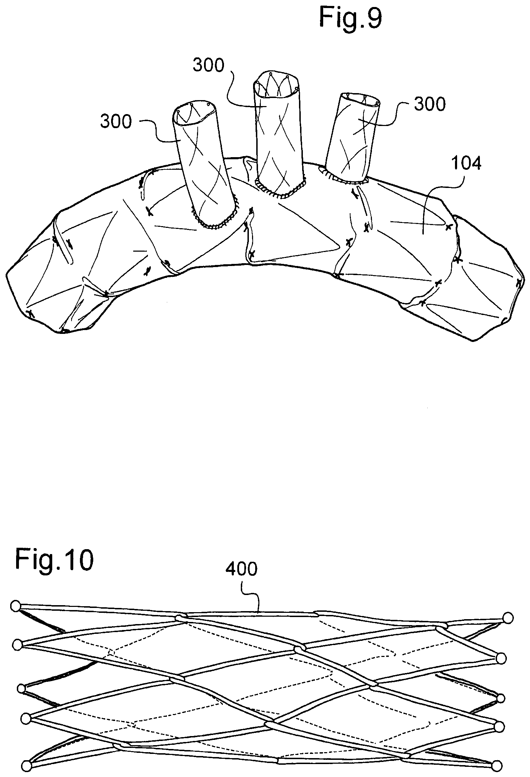

La figure 9 montre une vue en perspective d'une prothèse endovasculaire selon un mode de réalisation de l'invention. Chaque deuxième canal 300 est introduit dans un manchon 200 correspondant. Le pourtour extérieur d'un deuxième canal 300 épouse le pourtour intérieur du manchon 200 correspondant. Ceci fournit une bonne étanchéité. De cette manière la prothèse endovasculaire de l'invention gère des bifurcations de vaisseaux sanguins rencontrés dans l'anatomie d'un patient. Selon un mode de réalisation le manchon est réalisé en une matière tissée en polyester, ce qui offre une certaine marge d'extension du diamètre interne dudit manchon 200. Cette marge d'extension autorise une dilatation radiale du manchon 200 lors de l'introduction du deuxième canal 300 dans ledit manchon 200. Le manchon 200 se conforme autour du deuxième canal 300 pour maximiser l'étanchéité de la bifurcation.

En pratique il faut comprendre que le premier canal 100 doit idéalement combler un volume maximal à l'intérieur de vaisseau sanguin anévrismal. Pour cela, l'invention propose notamment que chaque manchon 200 s'étend à l'intérieur dudit canal 100, et ainsi ne gênant pas l'expansion maximale de celui-ci. Une bonne expansion du canal 100 permet d'épouser sensiblement la paroi interne du vaisseau sanguin et ainsi d'isoler et d'exclure la cavité anévrismale du flux sanguin.

Chaque manchon 200 est dépourvu d'armature métallique et pend librement à l'intérieur du canal 100. Le manchon ne comprend donc pas de structure rigide. Ceci offre une flexibilité de manipulation et notamment offre la possibilité d'utiliser un manchon en tant que point de bifurcation (voir figure 9) ou en tant que élément d'obturation.

La figure 10 représente vue de face d'une troisième armature expansible 400 de la prothèse endovasculaire selon un mode de réalisation de l'invention. Cette armature 400 est réalisée par tressage ou entrecroisement en un matériau analogue à la première armature expansible 102. Le maillage peut différencier par rapport à première armature expansible 102. Il est prévu que le diamètre de la troisième armature expansible 400 en état expansé est supérieur au diamètre de la première armature expansible 102 en état expansé et en conséquence supérieur au diamètre interne du premier canal 100.

Selon un mode de réalisation, la troisième armature expansible 400 est introduite à l'intérieur du premier canal 100 au niveau d'au moins un manchon 200.

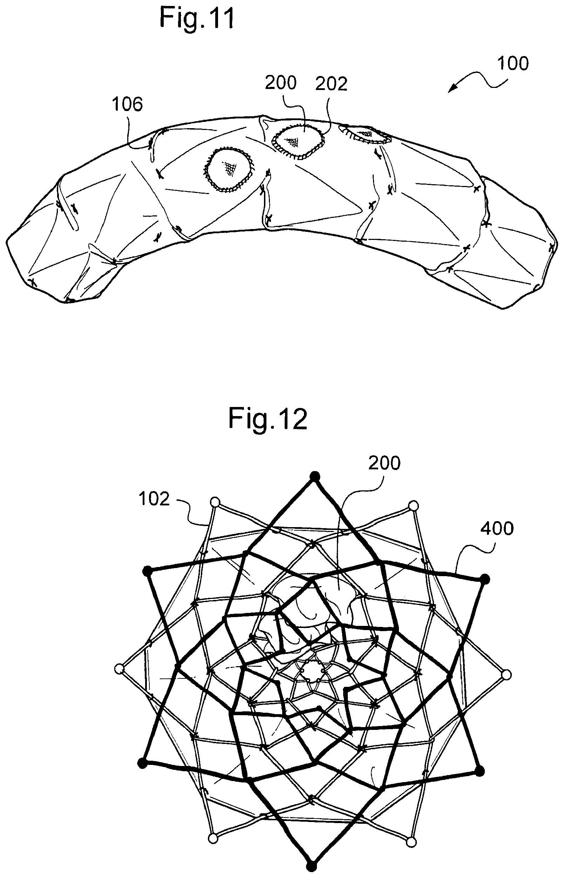

La figure 11 représente une vue en perspective d'une prothèse endovasculaire munie d'une troisième armature expansible et la figure 12 représente une vue de côté de la prothèse endovasculaire munie d'une troisième armature expansible.

Lors de l'introduction, la troisième armature expansible 400 est dans un état compressé. Lorsque la troisième armature expansible 400 est positionné au niveau d'au moins un manchon 200, elle s'étend et son diamètre augmente progressivement jusqu'à venir épouser la paroi interne du premier canal 100. Le manchon 200 s'étendant de manière souple à l'intérieur du premier canal 100 est alors plaqué contre la paroi interne du premier canal 100 en se pliant et formant ainsi une obturation de l'ouverture 112 de la première chemise. Dans ce cas le manchon 200 forme un opercule étanche pour les ouvertures 112.

Selon un mode de réalisation, le premier canal 100 présente une longueur de 30 à 250 mm et un diamètre de 10 à 50 mm. Chaque manchon 200 présente une longueur de 10 à 150 mm et un diamètre de 6 à 30 mm. Chaque deuxième canal 300 présente une longueur de 10 à 150 mm et un diamètre de 6 à 30 mm. Chaque troisième armature expansible 400 présente une longueur de 30 à 250 mm et un diamètre de 10 à 50 mm.

La prothèse endovasculaire de l'invention offre une liberté de manipulation au praticien hospitalier notamment grâce à sa double-fonctionnalité bifurcation/obturation. En effet, les techniques de positionnement de prothèse endovasculaire par cathéter sont parfois difficiles à mettre en œuvre pour des raisons d'anatomie. Lorsqu'une mise en place d'une bifurcation entre un premier canal 100 et un deuxième canal 300 pose des complications, l'invention permet d'étanchéifier le premier canal 100 par l'introduction d'une troisième armature expansible 400. La bifurcation et son irrigation sanguine (ou perfusion) peut ensuite être traitée séparément, par exemple par pontage.

L'invention vise également un procédé de mise en place d'une prothèse endovasculaire dans un patient.

Le procédé de l'invention comprend les étapes suivantes :
a) la mise à disposition d'une prothèse endovasculaire comprenant une première armature expansible et une première chemise pour ladite première armature, ladite première armature et ladite première chemise formant un premier canal lorsque la première armature est à l'état expansé, la première armature et la première chemise présentant chacune au moins une ouverture disposées sensiblement en regard l'une de l'autre et à travers lesquelles un manchon est reçu, ledit manchon présentant une première extrémité et une deuxième extrémité, et dont le pourtour de ladite première extrémité est solidaire avec le pourtour de l'ouverture de la première chemise tandis que ladite deuxième extrémité s'étend à l'intérieur dudit premier canal, et
b) le positionnement chez un individu de la prothèse endovasculaire mise à disposition à l'étape a).
Selon un mode de réalisation, le procédé de l'invention peut comprendre en outre les étapes suivantes :
c) la mise a disposition d'une deuxième armature expansible et d'une deuxième chemise pour ladite deuxième armature, ladite deuxième armature et ladite deuxième chemise formant un deuxième canal lorsque la deuxième armature est à l'état expansé, et
d) le positionnement dudit deuxième canal chez l'individu de l'étape b), ledit deuxième canal étant positionné pour déboucher dans ledit premier canal à travers un manchon correspondant, tandis que ce manchon épouse au moins partiellement le pourtour extérieur dudit deuxième canal pour former une bifurcation étanche.
Selon un autre mode de réalisation, le procédé de l'invention peut comprendre en outre les étapes suivantes :
e) la mise à disposition d'une troisième armature expansible, et
f) le positionnement de la troisième armature expansible chez l'individu de l'étape b), ladite troisième armature expansible étant positionnée à l'intérieur du premier canal au niveau des ouvertures à travers lesquelles un manchon est reçu, ladite troisième armature et ledit manchon formant un opercule pour lesdites ouvertures lorsque la troisième armature est à l'état expansé dans le premier canal.

Les positionnements auxquels est fait référence ci-dessus sont généralement réalisés par des méthodes d'angioplastie. Parmi ces méthodes on peut notamment citer les interventions chirurgicales par cathétérisme.

La présente invention a été essentiellement décrite en référence à l'anatomie humaine et spécifiquement à l'anatomie rencontrée au niveau de la crosse aortique. Toutefois, l'invention peut être utilisée dans d'autres parties du corps humain et peut également être utilisée chez tout autre mammifère.

L'invention propose ainsi une prothèse endovasculaire ou endoprothèse vasculaire comportant un premier élément extensible fait d'une armature rigide tapissée d'une membrane formé par un tissu synthétique. À l'état expansé, cet élément forme le canal principal de l'endoprothèse vasculaire. En plus de ses deux extrémités, ce premier canal ou « canal principal » comporte au moins une ouverture latérale de la membrane. Cette ouverture comporte un manchon de membrane (formé par le tissu synthétique) dirigé vers l'intérieur de la lumière du canal principal. Ce manchon peut-être appelé « manchon invaginé ». À l'ouverture latérale, la membrane du canal principal et celle du manchon invaginé sont en continuité absolue. À la différence du canal principal, le manchon invaginé ne comporte aucune armature rigide. L'ouverture latérale et le manchon invaginé du canal principal sont destinés à être positionnés en regard d'une collatérale vitale ou fonctionnelle pour recevoir une seconde endoprothèse vasculaire. La seconde endoprothèse vasculaire est formée par une armature rigide expansible tapissée d'une membrane et est destinée à s'étendre de la lumière du canal principal jusqu'aux premiers centimètres de la collatérale vitale ou fonctionnelle ou « canal secondaire ».

Le manchon invaginé assure non seulement la connexion et l'étanchéité entre le canal principal et le canal secondaire mais aussi, comme il sera décrit plus loin la, liberté de manipulation lors de la mise en place.

Selon l'anatomie à traiter, le canal principal peut comporter un ou plusieurs manchons invaginés pour recevoir un ou plusieurs canaux secondaires. Le canal principal et le canal secondaire comportent une armature rigide, par exemple métallique, tapissée d'une membrane étanche au sang et fine ayant démontré son aptitude à se substituer à la paroi artérielle chez l'humain. Les membranes les plus souvent retenues dans ce cadre sont faites de textile tissé ou tricoté de fibres de polyester ou d'équivalents non-tissés de poly-tetra-fluoro-éthylène ou PTFE. Le passage de la forme comprimée à la forme expansée repose sur l'aptitude de l'armature rigide à se déformer dans le domaine élastique, supra élastique ou plastique. L'épaisseur de la membrane est avantageusement inférieure à 0,4 mm pour une utilisation chez l'humain.

Le manchon invaginé peut être réalisé en utilisant la même membrane que celle du canal principal ou une membrane plus fine ou dont l'élasticité permet de donner à l'ensemble une meilleure étanchéité. Cette membrane utilisée pour le manchon invaginé est elle aussi apte à se substituer à la paroi artérielle chez l'humain. Un élément notable de l'invention est que le manchon invaginé n'est muni d'aucune armature rigide. À l'orifice de la membrane du canal principal, l'extrémité externe du manchon invaginé entre sensiblement en continuité avec celle de la membrane du canal principal. Cette continuité fourni non seulement la connexion mécanique mais également l'étanchéité. Elle peut être obtenue par suture, collage, soudure ou autre procédé ou encore par combinaison de procédés. L'extrémité interne du manchon invaginé est « libre » à l'intérieur de la lumière du canal principal ou tout au plus, fixée à la membrane de ce canal principal en un point afin de lui donner une direction.

La technique de mise en place du système en traitant une seule collatérale comporte notamment les étapes suivantes :
1) le canal principal est déployé de façon à ce que son extrémité proximale se situe sensiblement dans le collet proximal de l'anévrisme et que son extrémité distale se situe sensiblement dans le collet distal de l'anévrisme. L'orifice externe du manchon invaginé est positionné sensiblement en regard de l'ostium de la collatérale traitée dans l'anévrisme.
2) Le manchon invaginé est cathétérisé soit à partir de la collatérale traitée soit à partir de la lumière du canal principal à l'aide d'un guide métallique flexible.
3) Le canal secondaire est déployé après avoir été acheminé de façon coaxiale sur le guide de façon à ce que son extrémité proximale siège dans la lumière du canal principal et que son extrémité distale siège dans la lumière de la collatérale traitée, en zone saine. Le contact entre la membrane du manchon invaginé et celle du canal secondaire doit être suffisamment long et intime pour assurer une étanchéité parfaite entre les deux canaux. En revanche, le positionnement final et l'angle de raccordement entre les deux canaux dépendent de l'anatomie traitée.
4) En cas d'échec du cathétérisme du manchon invaginé, une certaine étanchéité de ce dernier est assurée par son collapsus (ou son repliement) du fait de la pression qui règne alors dans le canal principal. Ce canal principal se comporte alors comme une endoprothèse vasculaire classique. Cette situation peut être pérennisée par l'installation d'une seconde endoprothèse vasculaire classique de dimensions sensiblement correspondantes à celles du canal principal et déployée à l'intérieur de celui-ci plaquant le manchon invaginé sur sa face interne est fermant ainsi son orifice latéral. Dans ce cas, la perfusion (ou irrigation) de la collatérale vitale ou fonctionnelle est assurée par d'autres moyens, par exemple par un pontage. Cette étape de « sauvetage » a notamment pour but d'éviter une re-perfusion (re-irrigation) du sac anévrismal par la collatérale.

Les dimensions de la prothèse de l'invention dépendent sensiblement de l'anatomie traitée. Généralement, le canal principal est de diamètre constant ou non compris entre 10 et 50 mm, et de longueur comprise entre 30 et 250 mm. Généralement, le canal secondaire est de diamètre constant ou non compris entre 6 et 30 mm, et de longueur comprise entre 10 et 150 mm. Généralement, le manchon invaginé est de diamètre constant ou non compris entre 6 et 30 mm, et de longueur comprise entre 10 et 150 mm.

L'armature rigide est généralement métallique à déformation élastique. Les assemblages entre les différentes structures métalliques et les membranes (tissus synthétiques) sont réalisés par suture par fils synthétiques tressés tandis que la membrane de tous les éléments et du manchon est fait d'un tissage de polyester. Cependant, la construction des éléments peut faire appel à d'autres matériaux rigides et membraneux et à d'autres modes d'assemblage.

Dans un mode de réalisation particulier, l'invention peut également être définie de la manière suivante :
Endoprothèse vasculaire comprenant une première armature expansible et une première chemise pour ladite première armature, ladite première armature et ladite première chemise formant un premier canal lorsque la première armature est à l'état expansé, caractérisé en ce que la première armature et la première chemise présentent chacune au moins une ouverture disposées sensiblement en regard l'une de l'autre et à travers lesquelles un manchon souple est reçu, ledit manchon souple étant dépourvu d'armature et présentant une première extrémité et une deuxième extrémité, et dont le pourtour de ladite première extrémité est solidaire avec le pourtour de l'ouverture de la première chemise tandis que ladite deuxième extrémité s'étend à l'intérieur dudit premier canal.

Selon ce mode de réalisation, le kit de l'invention peut-être défini de la manière suivante:
Kit pour endoprothèse vasculaire comprenant :
   a) une première armature expansible et une première chemise pour ladite première armature, ladite première armature et ladite première chemise étant agencées pour former un premier canal lorsque la première armature est à l'état expansé,
   b) une deuxième armature expansible et une deuxième chemise pour ladite deuxième armature ladite deuxième armature et ladite deuxième chemise étant agencées pour former un deuxième canal lorsque la deuxième armature est à l'état expansé,
caractérisé en ce que la première armature expansible et la première chemise présentent chacune au moins une ouverture disposées sensiblement en regard l'une de l'autre et à travers lesquelles un manchon souple est reçu, ledit manchon ledit manchon souple étant dépourvu d'armature et présentant une première extrémité et une deuxième extrémité, et dont le pourtour de ladite première extrémité est solidaire avec le pourtour de l'ouverture de la première chemise tandis que ladite deuxième extrémité s'étend à l'intérieur dudit premier canal.

La caractéristique souple du manchon lui confère notamment une double fonction. Ainsi, dans une première position, le manchon rempli une fonction d'un point de bifurcation, tandis que dans une seconde position, le manchon rempli une fonction d'un élément d'obturation.

L'endoprothèse vasculaire et/ou le kit, tel(s) que défini(s) ci-dessus, comporte(nt) donc un manchon formant un point de bifurcation dans une première position et formant un élément d'obturation dans une seconde position.

## Revendications

1. Prothèse endovasculaire comprenant une première armature expansible (102) et une première chemise (104) pour ladite première armature, ladite première armature et ladite première chemise formant un premier canal (100) lorsque la première armature est à l'état expansé, **caractérisé en ce que** la première armature et la première chemise présentent chacune au moins une ouverture (110, 112) disposées sensiblement en regard l'une de l'autre et à travers lesquelles un manchon (200) est reçu, ledit manchon présentant une première extrémité (202) et une deuxième extrémité (204), et dont le pourtour de ladite première extrémité est solidaire avec le pourtour (114) de l'ouverture de la première chemise tandis que ladite deuxième extrémité s'étend à l'intérieur dudit premier canal (100), et **en ce que** chaque manchon (200) est dépourvu d'armature métallique et pend librement à l'intérieur du canal (100).

2. Prothèse endovasculaire selon la revendication 1, **caractérisé en ce que** la première armature expansible (102) et la première chemise (104) présentent chacune plusieurs ouvertures disposées sensiblement en regard les unes des autres et à travers lesquelles un manchon (200) est respectivement reçu, chaque manchon présentant une première extrémité (202) et une deuxième extrémité (204), et dont chaque pourtour des premières extrémités est solidaire avec chaque pourtour (114) des ouvertures de la première chemise tandis que chaque deuxième extrémité s'étend à l'intérieur dudit premier canal (100).

3. Prothèse endovasculaire selon l'une des revendications précédentes, **caractérisé en ce qu'**elle comprend en outre une deuxième armature expansible (302) et une deuxième chemise (304) pour ladite deuxième armature, ladite deuxième armature et ladite deuxième chemise formant un deuxième canal (300) lorsque la deuxième armature est à l'état expansé, et **en ce que** ledit deuxième canal (300) débouche dans ledit premier canal (100) à travers un manchon (200) correspondant, tandis que ce manchon (200) épouse au moins partiellement le pourtour extérieur dudit deuxième canal (300) pour former une bifurcation étanche.

4. Prothèse endovasculaire selon l'une des revendications précédentes, **caractérisé en ce qu'**elle comprend en outre une troisième armature expansible (400) disposée à 'l'intérieur du premier canal (100) au niveau des ouvertures à travers lesquelles un manchon (200) est reçu, ladite troisième armature expansible (400) et ledit manchon (200) formant un opercule pour lesdites ouvertures lorsque la troisième armature expansible (400) est à l'état expansé dans ledit premier canal (100).

5. Prothèse endovasculaire selon l'une des revendications précédentes, dans laquelle chaque armature (102, 302, 400) est réalisée en une matière métallique comprenant un acier inoxydable biocompatible, et de préférence comprenant un matériau radio-opaque.

6. Prothèse endovasculaire selon l'une des revendications 1 à 5, dans laquelle chaque chemise (104, 304) est réalisée en une matière tissée, de préférence une matière tissée en polyester.

7. Prothèse endovasculaire selon l'une des revendications des revendications 1 à 5, dans laquelle chaque chemise (104, 304) comprend une membrane poreuse, de préférence une membrane en poly-tetra-fluoro-éthylène.

8. Prothèse endovasculaire selon l'une des revendications précédentes, dans laquelle chaque manchon (200) est réalisé en une matière sensiblement identique à celle de chaque chemise (104, 304).

9. Prothèse endovasculaire selon l'une des revendications précédentes, dans laquelle ledit premier canal (100) présente une longueur comprise entre 30mm et 250mm et un diamètre compris entre 10mm et 50mm, et dans laquelle chaque manchon (200) présente une longueur comprise entre 10mm et 150mm et un diamètre compris entre 6mm et 20mm.

10. Prothèse endovasculaire selon l'une des revendications précédentes, dans laquelle chaque chemise (104, 304) présente une épaisseur inférieure ou égale à 0,4 millimètre.

11. Prothèse endovasculaire selon l'une des revendications précédentes, dans laquelle la première armature expansible (102) comprend une grille métallique expansible, et dans laquelle la première chemise (104) est fixée à cette grille métallique expansible par des points de couture (106).

12. Prothèse endovasculaire selon l'une des revendications précédentes, dans laquelle chaque pourtour de chaque première extrémité (202) de manchon (200) est solidarisé par couture respectivement avec chaque pourtour (114) d'ouverture de la première chemise correspondant.

13. Kit pour prothèse endovasculaire comprenant :
a) une première armature expansible (102) et une première chemise (104) pour ladite première armature, ladite première armature et ladite première chemise étant agencées pour former un premier canal (100) lorsque la première armature est à l'état expansé,
b) une deuxième armature (302) expansible et une deuxième chemise (304) pour ladite deuxième armature ladite deuxième armature et ladite deuxième chemise étant agencées pour former un deuxième canal (300) lorsque la deuxième armature est à l'état expansé,
**caractérisé en ce que** la première armature expansible (102) et la première chemise (104) présentent chacune au moins une ouverture disposées sensiblement en regard l'une de l'autre et à travers lesquelles un manchon (200) est reçu, ledit manchon (200) présentant une première extrémité (202) et une deuxième extrémité (204), et dont le pourtour de ladite première extrémité (202) est solidaire avec le pourtour (114) de l'ouverture de la première chemise (104) tandis que ladite deuxième extrémité (204) s'étend à l'intérieur dudit premier canal (100), et **en ce que** chaque manchon (200) est dépourvu d'armature métallique et pend librement à l'intérieur du canal (100).

14. Kit selon la revendication 13, comprenant en outre une troisième armature expansible (400) prévue pour être disposée à l'intérieur dudit premier canal (100) au niveau des ouvertures (110, 112) à travers lesquelles un manchon (200) est reçu.

## Patentansprüche

1. Endovaskuläre Prothese, umfassend ein erstes ausdehnbares Gerüst (102) und eine erste Hülle (104) für das erste Gerüst, wobei das erste Gerüst und die erste Hülle einen ersten Kanal (100) bilden, wenn das erste Gerüst im ausgedehnten Zustand ist, **dadurch gekennzeichnet, dass** das erste Gerüst und die erste Hülle jeweils mindestens eine Öffnung (110, 112) aufweisen, die im Wesentlichen gegenüberliegend angeordnet sind und durch die ein Stutzen (200) aufgenommen wird, wobei der Stutzen ein erstes Ende (202) und ein zweites Ende (204) aufweist und wobei der Umfang des ersten Endes fest mit dem Umfang (114) der Öffnung der ersten Hülle verbunden ist, während das zweite Ende sich in das Innere des ersten Kanals (100) erstreckt, und dass jeder Stutzen (200) frei von metallischem Gerüst ist und frei im Inneren des Kanals (100) hängt.

2. Endovaskuläre Prothese nach Anspruch 1, **dadurch gekennzeichnet, dass** das erste ausdehnbare Gerüst (102) und die erste Hülle (104) jeweils mehrere Öffnungen aufweisen, die im Wesentlichen gegenüberliegend angeordnet sind und durch die jeweils ein Stutzen (200) aufgenommen wird, wobei jeder Stutzen ein erstes Ende (202) und ein zweites Ende (204) aufweist und wobei jeder Umfang der ersten Enden fest mit jedem Umfang (114) der Öffnungen der ersten Hülle verbunden ist, während jedes zweite Ende sich in das Innere des ersten Kanals (100) erstreckt.

3. Endovaskuläre Prothese nach einem der vorhergehenden Ansprüche, **dadurch gekennzeichnet, dass** sie weiterhin ein zweites ausdehnbares Gerüst (302) und eine zweite Hülle (304) für das zweite Gerüst umfasst, wobei das zweite Gerüst und die zweite Hülle einen zweiten Kanal (300) bilden, wenn das zweite Gerüst im ausgedehnten Zustand ist, und dass der zweite Kanal (300) in dem ersten Kanal (100) durch einen entsprechenden Stutzen (200) mündet, während dieser Stutzen (200) sich zumindest zum Teil an den Außenumfang des zweiten Kanals (300) anschmiegt, um eine dichte Gabelung zu bilden.

4. Endovaskuläre Prothese nach einem der vorhergehenden Ansprüche, **dadurch gekennzeichnet, dass** sie weiterhin ein drittes ausdehnbares Gerüst (400) umfasst, das im Inneren des ersten Kanals (100) auf der Ebene von Öffnungen, durch die ein Stutzen (200) aufgenommen wird, angeordnet ist, wobei das dritte ausdehnbare Gerüst (400) und der Stutzen (200) ein Verschlussorgan für die Öffnungen bilden, wenn das dritte ausdehnbare Gerüst (400) in dem ersten Kanal (100) im ausgedehnten Zustand ist.

5. Endovaskuläre Prothese nach einem der vorhergehenden Ansprüche, wobei jedes Gerüst (102, 302, 400) aus einem metallischen Material hergestellt ist, das einen biokompatiblen Edelstahl umfasst und vorzugsweise ein röntgendichtes Material umfasst.

6. Endovaskuläre Prothese nach einem der Ansprüche 1 bis 5, wobei jede Hülle (104, 304) aus einem Gewebematerial, vorzugsweise einem Gewebematerial aus Polyester hergestellt ist.

7. Endovaskuläre Prothese nach einem der Ansprüche 1 bis 5, wobei jede Hülle (104, 304) eine poröse Membran, vorzugsweise eine Membran aus Polytetrafluorethylen umfasst.

8. Endovaskuläre Prothese nach einem der vorhergehenden Ansprüche, wobei jeder Stutzen (200) aus einem Material hergestellt ist, das im Wesentlichen mit dem jeder Hülle (104, 304) identisch ist.

9. Endovaskuläre Prothese nach einem der vorhergehenden Ansprüche, wobei der erste Kanal (100) eine Länge, die zwischen 30 mm und 250 mm liegt, und einen Durchmesser, der zwischen 10 mm und 50 mm liegt, aufweist und wobei jeder Stutzen (200) eine Länge, die zwischen 10 mm und 150 mm liegt, und einen Durchmesser, der zwischen 6 mm und 20 mm liegt, aufweist.

10. Endovaskuläre Prothese nach einem der vorhergehenden Ansprüche, wobei jede Hülle (104, 304) eine Dicke aufweist, die kleiner als oder gleich 0,4 Millimeter ist.

11. Endovaskuläre Prothese nach einem der vorhergehenden Ansprüche, wobei das erste ausdehnbare Gerüst (102) ein ausdehnbares metallisches Gitter umfasst und wobei die erste Hülle (104) an diesem ausdehnbaren metallischen Gitter durch Nahtstellen (106) befestigt ist.

12. Endovaskuläre Prothese nach einem der vorhergehenden Ansprüche, wobei jeder Umfang von jedem ersten Ende (202) des Stutzens (200) durch eine Naht jeweils fest mit jedem entsprechenden Umfang (114) einer Öffnung der ersten Hülle verbunden wird.

13. Kit für eine endovaskuläre Prothese, umfassend:
a) ein erstes ausdehnbares Gerüst (102) und eine erste Hülle (104) für das erste Gerüst, wobei das erste Gerüst und die erste Hülle dazu eingerichtet sind, einen ersten Kanal (100) zu bilden, wenn das erste Gerüst im ausgedehnten Zustand ist,
b) ein zweites ausdehnbares Gerüst (302) und eine zweite Hülle (304) für das zweite Gerüst, wobei das zweite Gerüst und die zweite Hülle dazu eingerichtet sind, einen zweiten Kanal (300) zu bilden, wenn das zweite Gerüst im ausgedehnten Zustand ist,
**dadurch gekennzeichnet, dass** das erste ausdehnbare Gerüst (102) und die erste Hülle (104) jeweils mindestens eine Öffnung aufweisen, die im Wesentlichen gegenüberliegend angeordnet sind und durch die ein Stutzen (200) aufgenommen wird, wobei der Stutzen (200) ein erstes Ende (202) und ein zweites Ende (204) aufweist und wobei der Umfang des ersten Endes (202) fest mit dem Umfang (114) der Öffnung der ersten Hülle (104) verbunden ist, während das zweite Ende (204) sich in das Innere des ersten Kanals (100) erstreckt, und dass jeder Stutzen (200) frei von metallischem Gerüst ist und frei im Inneren des Kanals (100) hängt.

14. Kit nach Anspruch 13, weiterhin umfassend ein drittes ausdehnbares Gerüst (400), das vorgesehen ist, um im Inneren des ersten Kanals (100) auf der Ebene von den Öffnungen (110, 112), durch die ein Stutzen (200) aufgenommen wird, angeordnet zu werden.

## Claims

1. Endovascular prosthesis comprising a first expandable framework (102) and a first jacket (104) for said first framework, said first framework and said first jacket forming a first channel (100) when the first framework is in the expanded state, **characterised in that** the first framework and the first jacket each have at least one opening (110, 112), which openings are arranged substantially opposite one another and through which a sleeve (200) is received, said sleeve having a first end (202) and a second end (204), the perimeter of said first end being attached to the perimeter (114) of the opening of the first jacket, while said second end extends inside said first channel (100), and **in that** each sleeve (200) does not have a metallic framework and hangs freely inside the channel (100).

2. Endovascular prosthesis according to claim 1, **characterised in that** the first expandable framework (102) and the first jacket (104) each have a plurality of openings which are arranged substantially opposite one another and through which a sleeve (200) is respectively received, each sleeve having a first end (202) and a second end (204), and each perimeter of the first ends being attached to each perimeter (114) of the openings of the first jacket, while each second end extends inside said first channel (100).

3. Endovascular prosthesis according to any one of the preceding claims, **characterised in that** it further comprises a second expandable framework (302) and a second jacket (304) for said second framework, said second framework and said second jacket forming a second channel (300) when the second framework is in the expanded state, and **in that** said second channel (300) opens into said first channel (100) through a corresponding sleeve (200), while the sleeve (200) fits at least partially the outer perimeter of said second channel (300) to form a leak-tight bifurcation.

4. Endovascular prosthesis according to any one of the preceding claims, **characterised in that** it further comprises a third expandable framework (400) arranged inside the first channel (100) at the openings through which a sleeve (200) is received, said third expandable framework (400) and said sleeve (200) forming an operculum for said openings when the third expandable framework (400) is in the expanded state inside said first channel (100).

5. Endovascular prosthesis according to any one of the preceding claims, wherein each framework (102, 302, 400) is made of a metallic material comprising a biocompatible stainless steel, and preferably comprising a radio-opaque material.

6. Endovascular prosthesis according to any one of claims 1 to 5, wherein each jacket (104, 304) is made of a woven material, preferably of a polyester woven material.

7. Endovascular prosthesis according to any one of claims 1 to 5, wherein each jacket (104, 304) comprises a porous membrane, preferably a membrane of polytetrafluoroethylene.

8. Endovascular prosthesis according to any one of the preceding claims, wherein each sleeve (200) is made of a material which is substantially identical to that of each jacket (104, 304).

9. Endovascular prosthesis according to any one of the preceding claims, wherein said first channel (100) has a length of from 30 mm to 250 mm and a diameter of from 10 mm to 50 mm, and wherein each sleeve (200) has a length of from 10 mm to 150 mm and a diameter of from 6 mm to 20 mm.

10. Endovascular prosthesis according to any one of the preceding claims, wherein each jacket (104, 304) has a thickness of less than or equal to 0.4 millimetre.

11. Endovascular prosthesis according to any one of the preceding claims, wherein the first expandable framework (102) comprises an expandable metal grating, and wherein the first jacket (104) is fixed to the expandable metallic grating by stitches (106).

12. Endovascular prosthesis according to any one of the preceding claims, wherein each perimeter of each first end (202) of a sleeve (200) is attached by stitching to each corresponding perimeter (114) of an opening of the first jacket.

13. Kit for an endovascular prosthesis, comprising:
a) a first expandable framework (102) and a first jacket (104) for said first framework, said first framework and said first jacket being arranged to form a first channel (100) when the first framework is in the expanded state,
b) a second expandable framework (302) and a second jacket (304) for said second framework, said second framework and said second jacket being arranged to form a second channel (300) when the second framework is in the expanded state,
**characterised in that** the first expandable framework (102) and the first jacket (104) each have at least one opening, which openings are arranged substantially opposite one another and through which a sleeve (200) is received, said sleeve (200) having a first end (202) and a second end (204), the perimeter of said first end (202) being attached to the perimeter (114) of the opening of the first jacket (104), while said second end (204) extends inside said first channel (100), and **in that** each sleeve (200) does not have a metallic framework and hangs freely inside the channel (100).

14. Kit according to claim 13, further comprising a third expandable framework (400) provided to be arranged inside said first channel (100) at the openings (110, 112) through which a sleeve (200) is received.
